(19) Europäisches Patentamt
European Patent Office
Office européen des brevets

(11) **EP 3 469 245 B1**

(12) **EUROPÄISCHE PATENTSCHRIFT**

(45) Veröffentlichungstag und Bekanntmachung des Hinweises auf die Patenterteilung:
**27.09.2023 Patentblatt 2023/39**

(21) Anmeldenummer: **17734225.0**

(22) Anmeldetag: **27.04.2017**

(51) Internationale Patentklassifikation (IPC):
**F16L 33/02** *(2006.01)* **A61M 39/12** *(2006.01)*
**B65D 63/10** *(2006.01)* **H02G 3/32** *(2006.01)*

(52) Gemeinsame Patentklassifikation (CPC):
**F16L 33/02; A61M 39/12; B65D 63/1027; H02G 3/32; B65D 2563/108**

(86) Internationale Anmeldenummer:
**PCT/DE2017/000115**

(87) Internationale Veröffentlichungsnummer:
**WO 2017/211334 (14.12.2017 Gazette 2017/50)**

(54) **KABELBINDER**

CABLE TIE

ATTACHE-CÂBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.06.2016 DE 102016007091**

(43) Veröffentlichungstag der Anmeldung:
**17.04.2019 Patentblatt 2019/16**

(73) Patentinhaber: **Xenios AG**
**74076 Heilbronn (DE)**

(72) Erfinder: **BEURER, Matthias**
**70469 Stuttgart (DE)**

(74) Vertreter: **Graf von Stosch, Andreas**
**Graf von Stosch**
**Patentanwaltsgesellschaft mbH**
**Prinzregentenstraße 22**
**80538 München (DE)**

(56) Entgegenhaltungen:
**EP-B1- 2 247 882      WO-A1-00/00407**
**US-A- 381 426      US-A- 3 197 164**
**US-A- 5 690 522**

EP 3 469 245 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft einen Kabelbinder und ein Set mit einem derartigen Kabelbinder.

Kabelbinder werden auch als Kabelband oder Ratsch-Band bezeichnet und bilden ein universell einsetzbares, schnell zu verwendendes und preisgünstiges Verbindungselement.

Ursprünglich wurden Kabelbinder dafür entwickelt, verschiedene Kabel und ganze Kabelbäume aneinander oder an anderen Gegenständen zu fixieren. Kabelbinder werden jedoch auch im Baugewerbe zur provisorischen Montage, als Verpackungshilfsmittel oder in der polizeilichen Praxis zur Fesselung von Menschen verwendet. Die Erfindung betrifft auch spezielle Kabelbinder mit angespritzten Befestigungselementen wie Klebesockeln, Steckankern oder Lamellenfüßen, wie sie beispielsweise im Automobilbereich verwendet werden.

Erfindungsgemäße Kabelbinder werden aus Kunststoffen, insbesondere aus PA 6.6 oder PVC gefertigt. Polyamid 6.6, das auch als Nylon® bekannt ist, hat sich für Kabelbinder als besonders vorteilhaft herausgestellt. Die Kabelbinder können jedoch auch aus anderen Materialien, wie beispielsweise Polyurethan, POM, ABS, PP, PE, PC, PEEK, anderen Kunststoffen, Metall, Aluminiumguss bis hin zu Edelstahl hergestellt werden.

Bei Einwegkabelbindern wird durch eine Innen- oder Außenverzahnung erreicht, dass sich der Kabelbinder nicht mehr öffnen lässt. Er kann normalerweise nur durch Zerstörung wieder geöffnet werden. Bei Kabelbindern mit einer Sperrzunge aus Kunststoff ist es meist möglich, diese mit einer Nadel zu entriegeln und die Schlaufe so wieder zu öffnen. Um Kabelbinder mit einer besonders hohen Zugkraft herzustellen, wird in den Kopf eine Metallzunge eingearbeitet, die es ermöglicht, dass der Kabelbinder durch Einschneiden der Zunge in den Kabelbinderkörper verschließt.

Mehrwegkabelbinder können durch eine Entriegelung wieder geöffnet und so mehrfach verwendet werden. Daneben gibt es auch Kabelbinder mit offenem Binderkopf, Doppelkopf-Kabelbinder und Kabelbinder mit besonderem Beschriftungsfeld.

Die Erfindung betrifft alle derartigen Kabelbinder und insbesondere den Einsatz von Kabelbindern zur Befestigung eines Schlauches an einem Rohrstück. Diese Befestigung ist in der Medizintechnik weit verbreitet, um Leitungen für Flüssigkeiten, wie insbesondere Blut oder Gase, an einem Schlauchkonnektor, d.h. einem Rohr oder einem Rohrstutzen zu befestigen. Dabei werden die aus flexiblem Material hergestellten Schläuche mittels des Kabelbinders derart an das Rohrstück gepresst, dass eine dichte Verbindung entsteht.

Insbesondere bei diesem Einsatzgebiet entsteht das Problem, dass das Schlauchmaterial durch Alterung oder Sterilisation geringfügig schrumpfen kann oder seine elastischen Eigenschaften verliert. Dadurch verringert sich der Anpressdruck zwischen dem Schlauch und dem Rohrstück und dies kann zu Dichtigkeitsproblemen führen. In der Praxis entstehen die ersten Undichtheiten meistens im Bereich des Kopfes des Kabelbinders, in dem der Kabelbinder nicht flächig am Schlauch anliegt. Daher wurde vorgeschlagen, den Kabelbinderkopf so zu formen, dass der Kabelbinder auf einem möglichst großen Umfangsflächenbereich am Schlauch anliegt.

Diese Kabelbinder haben jedoch den Nachteil, dass für jeden Schlauchdurchmesser, ein spezieller Kabelbinder bereitgestellt werden muss. Dadurch steigt der Aufwand für die Herstellung eines einzelnen Kabelbinders und für die meist im Spritzgussverfahren hergestellten Kabelbinder, müssen unterschiedliche Formen bereitgestellt werden.

Kabelbinder mit einem Kopfstück und einer Lasche, die in das Kopfstück geschoben werden kann und sich dabei fixiert, wobei ein Ausgleichsstück an der Lasche befestigt ist, sind beispielsweise aus der EP 2 247 882 B1 bekannt.

Der Erfindung liegt daher die Aufgabe zugrunde, einen Kabelbinder derart weiterzuentwickeln, dass er einen sicheren Anpressdruck bereitstellt, der einen durch den Kabelbinder zusammen gepressten Gegenstand möglichst gleichmäßig am Umfang verformt.

Diese Aufgabe wird mit einem Kabelbinder mit den Merkmalen des Patentanspruchs 1 gelöst.

Erfindungsgemäß wird somit vorgeschlagen, einen bekannten Kabelbinder mit einem Ausgleichsstück zu kombinieren, das auf den Kabelbinder aufgefädelt wird und das eine einen langen Schenkel bildende Fläche aufweist, die als Anlage für das Kopfstück plan ausgebildet ist. Dieses Ausgleichsstück erlaubt es einerseits, in großen Stückzahlen hergestellte bekannte billige Kabelbinder zu verwenden, und andererseits, einen Kabelbinder bereitzustellen, der optimal an einen speziellen Einsatzzweck angepasst ist.

Das Ausgleichsstück weist in der Ebene der Umgrenzung des Hohlraumes einen keilförmigen Querschnitt mit zwei langen Schenkeln und einer kurzen Basis auf. Die zwei langen Schenkel können somit einen Keil zwischen dem Kopfstück und dem umschlossenen Hohlraum bilden, während die kurze Basis es erleichtert, das Ausgleichsstück auf der Lasche zu verschieben. Die Lasche erstreckt sich dann quer zu den langen Schenkeln, verschiebbar in einem Durchbruch im Ausgleichsstück und dann durch die Öffnung im Kopfstück.

Dabei ist eine einen langen Schenkel bildende Fläche als Anlage für das Kopfstück plan ausgebildet. Dadurch wird eine Anlagefläche gebildet, die auch eine geringfügige Verschieblichkeit der Flächen zueinander ermöglicht.

Derartige Ausgleichsstücke sind kostengünstig herstellbar und mit bekannten Kabelbindern kombinierbar. Dies ermöglicht es insbesondere, günstige, im Spritzgussverfahren hergestellte Standardkabelbinder mit einem individuellen Ausgleichsstück zu kombinieren, das durch die Formgebung, die Materialwahl und/oder die Farbe optimal an einen speziellen Einsatzzweck angepasst ist.

Das Ausgleichsstück kann an einer beliebigen Stelle auf der Lasche des Kabelbinders positioniert werden. Es liegt bei der Verwendung des Kabelbinders am Kopfstück des Kabelbinders an. Die Zweiteiligkeit von Kopfstück und Ausgleichsstück führt dazu, dass das Ausgleichsstück beim Anziehen des Kabelbinders optimal positioniert wird und bei Erschütterungen oder einer Formveränderung der Materialien, sich die Position des Ausgleichsstücks auf der Lasche oder relativ zum Kopfstück geringfügig verändern kann. Dadurch kann der Anpressdruck länger erhalten bleiben.

Vorteilhafte Ausgestaltungen sind Gegenstand der Unteransprüche.

Insbesondere für das Anpressen eines Schlauches an einem Rohrstück wird vorgeschlagen, dass das Ausgleichsstück, an seiner dem Hohlraum zugeordneten Seite, eine konkave Oberfläche aufweist. Eine derartige konkave Oberfläche kann den "Zwickelraum" zwischen Kopfstück und Lasche ausfüllen, um die Anlagefläche zu vergrößern. Je nach Einsatzzweck können an Stelle einer konkaven Oberfläche auch eine plane Fläche oder eine strukturierte Oberfläche vorgesehen werden, da sich ein elastisches Schlauchmaterial an die Form der Oberfläche anpasst.

Wenn die Lasche einen kreisförmigen Hohlraum umgrenzt, kann die konkave Oberfläche des Ausgleichsstücks etwa dem Radius des kreisförmigen Hohlraumes entsprechen. Etwa heißt in diesem Zusammenhang, eine Abweichung der Radien des Ausgleichsstücks vom Radius des umgrenzten Hohlraumes von maximal +/- 30 % und vorzugsweise von maximal +/- 10 % und besonders bevorzugt von maximal +/- 1 %.

Ein in der Medizintechnik besonders häufiger Einsatzzweck liegt in der Befestigung von Schläuchen mit einem Durchmesser von $\frac{3}{8}$ Zoll und $\frac{1}{4}$ Zoll. Daher wird vorgeschlagen, dass die konkave Oberfläche etwa der Oberfläche eines Schlauches mit einem Durchmesser von $\frac{3}{8}$ Zoll oder $\frac{1}{4}$ Zoll entspricht.

Die konkave Oberfläche grenzt auf ihrer dem Hohlraum zugewandten Seite einerseits an die Lasche und andererseits an das Kopfstück an. Ein Absatz, der beispielsweise 0,5 mm bis 5,0 mm aufweist, kann entweder auf einer dieser Seiten oder auf beiden Seiten vorgesehen sein. Dieser Absatz erleichtert die Fertigung des Ausgleichsstücks und durch den Absatz, der auch gerundet ausgebildet werden kann, werden scharfe Kanten vermieden, die bei unsachgemäßer Verwendung als Schneidefläche einen Schlauch beschädigen könnten.

Die Handhabbarkeit des Ausgleichsstückes wird verbessert, wenn der Übergang von einer einen langen Schenkel bildenden Fläche zu einer die Basis bildenden Fläche abgerundet ist. Vorzugsweise sind beide Übergangsflächen leicht abgerundet.

Eine nicht beanspruchte Ausführungsform sieht vor, dass die Anlagefläche so ausgebildet ist, dass während der Anlage eine Zentrierung der Flächen zueinander bewirkt wird. Dies wird beispielsweise durch eine konkave Fläche erreicht, die in eine konvexe Fläche passend eingreift. Außerdem kann an dieser Anlagefläche zwischen dem Kopfstück und dem Ausgleichsstück eine Struktur vorgesehen sein, die die Verschieblichkeit der Flächen relativ zueinander einschränkt.

Ein leichtes Auffädeln des Ausgleichsstückes auf die Lasche wird dadurch erzielt, dass das Ausgleichsstück einen rechteckigen Schlitz für die Aufnahme der Lasche aufweist, dessen längere Seite der Breite der Lasche entspricht, sodass es auf der Lasche verschiebbar ist und nicht lose auf der Lasche rutscht. Das Ausgleichsstück ist somit mittels eines leichten Druckes auf der Lasche verschiebbar und rutscht durch sein Eigengewicht nicht selbstständig von der Lasche.

Besonders vorteilhaft ist es, wenn das Ausgleichsstück einen rechteckigen Schlitz für die Aufnahme der Lasche aufweist, dessen kürzere oder längere Seite eine federnd oder elastisch ausgebildete Anlagefläche aufweist, sodass diese Anlagefläche gegen die Lasche drückt. Dies stellt sicher, dass auch bei wiederholter Verwendung des Ausgleichsstückes, das Ausgleichsstück nicht unbeabsichtigt von der Lasche rutscht.

Das Ausgleichsstück kann einen Anschlag aufweisen, der mit einem Gegenstück an der Lasche zusammenwirkt. Dieser Anschlag sorgt dafür, dass das Ausgleichsstück nur bis zu einer bestimmten Position auf die Lasche aufgeschoben werden kann. Dieser Anschlag kann beispielsweise auch Teil einer Rasteinrichtung im Ausgleichsstück sein, sodass die Position am Anschlag fühlbar verändert werden kann.

Da sich im Laufe der Zeit die Lasche des Kabelbinders verlängern kann, die Rasteinrichtung im Kopfstück nachgeben kann oder der von der Lasche umgebene Gegenstand, wie beispielsweise eine Schlauchleitung schrumpfen kann, ist es vorteilhaft, wenn auch in diesem Fall ein fester Anpressdruck erhalten bleibt. Dies wird dadurch erreicht, dass das Ausgleichsstück ein elastisches Teil ist oder aufweist, das sich unter Druck verformt. Dadurch kann beispielsweise erreicht werden, dass das Ausgleichsstück auch bei einem um etwa einen oder mehrere Millimeter im Durchmesser schrumpfenden Schlauch weiterhin am Schlauch anliegt. Somit muss die Elastizität nicht an der Lasche oder am Kopfstück vorgesehen sein, sondern eine Elastizität an einem Teil des Ausgleichsstückes oder ein Ausgleichsstück aus einem elastischen Material, können die notwendige Elastizität bereitstellen, um einen ausreichenden Anpressdruck von der Lasche an das von der Lasche umgebenen Material aufrechtzuerhalten.

Das Ausgleichsstück kann hierfür insbesondere an der dem Hohlraum zugewandten Seite eine elastische Anlagefläche aufweisen, die ein Druckpolster bildet. Das Ausgleichsstück kann jedoch auch aus einem elastischeren Material als das Kopfstück mit der Lasche ausgebildet sein, um die Federwirkung zu erzielen.

Die Elastizität kann auch durch eine spezielle Struktur an den Anlageflächen der Lasche erzielt werden. Hierfür können beispielsweise Zacken oder Riffelungen vorgesehen werden, die sich auf Druck elastisch verbiegen.

Um einen Kabelbinder für spezielle Schlauchdurchmesser optimiert herzustellen, wird vorgeschlagen, ein Set aus einem derartigen Kabelbinder und einem Schlauchstück bereitzustellen oder spezielle Schlauchstücke mit einem speziell auf sie abgestimmten Kabelbinder mit Ausgleichsstück anzubieten.

Das Ausgleichsstück kann als Spritzgussteil und vorzugsweise als Zweikomponentenspritzgussteil hergestellt sein. Das Zweikomponentenspritzgussteil ermöglicht es, funktional unterschiedliche Bereiche, wie federnde Anlageflächen und stabile Formgebungsbereiche während eines Spritzgussvorganges aus unterschiedlichem Material herzustellen. Für die Herstellung von kleineren Stückzahlen und für die Multikomponenten-Herstellung wird die Verwendung eines 3D-Druckverfahrens vorgeschlagen.

Bei einem im Querschnitt keilförmigen Ausgleichsstück kann der kürzere Schenkel ein Etikett oder einen Aufdruck aufweisen. Außerdem können unterschiedliche Ausgleichsstücke in unterschiedlichen Farben hergestellt werden.

Unterschiedliche Ausführungsvarianten an Kabelbindern und Ausgleichsstücken sind in der Zeichnung dargestellt und werden im Folgenden näher erläutert. Es zeigt

Figur 1    schematisch einen Schnitt durch einen bekannten Kabelbinder,

Figur 2    eine perspektivische Ansicht eines Kabelbinders mit Ausgleichsstück,

Figur 3    einen Schnitt durch einen Kabelbinder mit Ausgleichsstück,

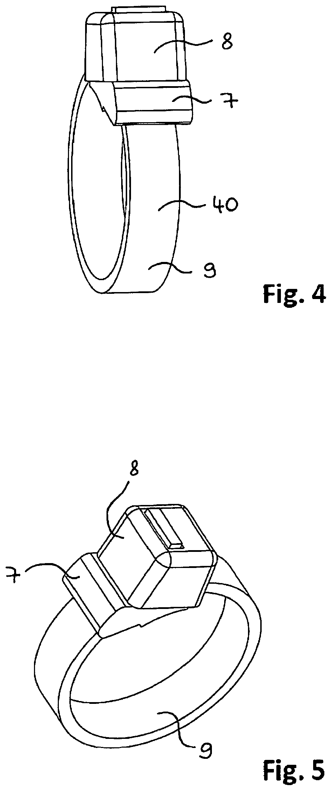

Figur 4    eine erste perspektivische Ansicht des in Figur 3 gezeigten Kabelbinders,

Figur 5    eine zweite perspektivische Ansicht des in Figur 3 gezeigten Kabelbinders,

Figur 6    das in Figur 3 gezeigte Ausgleichsstück als Detail,

Figur 7    teilweise geschnitten einen Kabelbinder mit einem Ausgleichsstück, das eine flexible Anlagefläche aufweist,

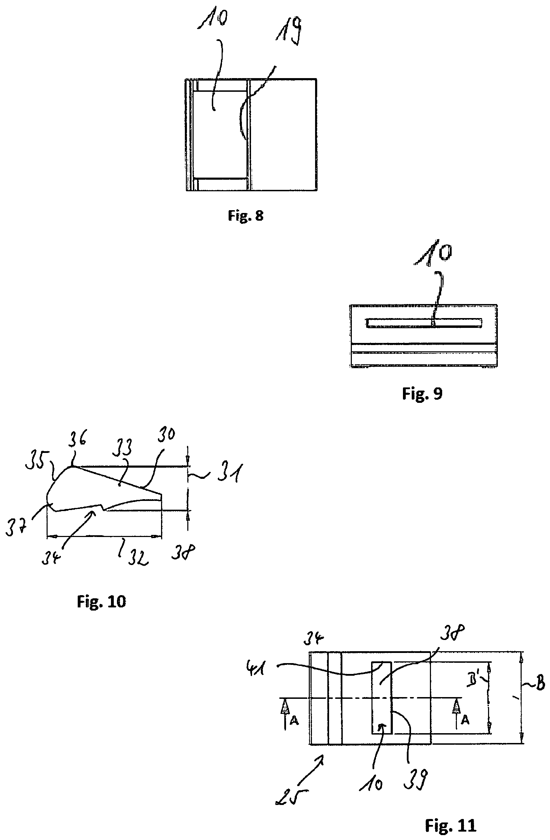

Figur 8    eine Vorderansicht auf das in Figur 7 gezeigte Ausgleichsstück,

Figur 9    eine Rückansicht des in Figur 7 gezeigten Ausgleichsstückes,

Figur 10    eine Seitenansicht des in Figur 7 gezeigten Ausgleichsstückes,

Figur 11    eine Draufsicht auf das in Figur 7 gezeigte Ausgleichsstück,

Figur 12    eine Ansicht eines Schnittes durch das in Figur 7 gezeigte Ausgleichsstück,

Figur 13    eine perspektivische Ansicht eines Ausgleichsstückes gemäß Figur 7 mit angedeuteter elastischer Auflage und

Figur 14    ein Ausgleichsstück gemäß Figur 7 mit angedeuteter bedruckbarer Platte.

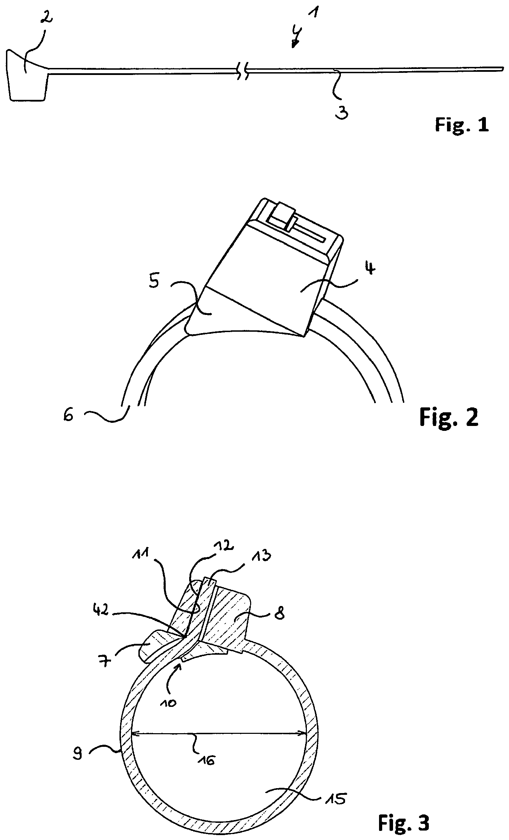

Der in der Figur 1 gezeigte Kabelbinder 1 besteht aus einem Kopfstück 2 und einer Lasche 3.

Bei dem in Figur 2 gezeigtem Ausführungsbeispiel liegt am Kopfstück 4 ein Ausgleichsstück 5 an und die Lasche 6 ist durch das Ausgleichsstück 5 und das Kopfstück 4 gezogen. Eine ähnliche Ausbildung zeigt das Ausführungsbeispiel gemäß Figur 3. Hier liegt das Ausgleichsstück 7 am Kopfstück 8 an und die Lasche 9 ist durch eine Öffnung 10 im Ausgleichsstück 7 und durch das Kopfstück 8 gefädelt. Eine Verzahnung 11 am Kopfstück 8 wirkt mit einer Verzahnung 12 an der Lasche 9 zusammen, um das Ende 13 der Lasche 9 im Kopf 8 zu halten.

Außerdem kann die Kante 14 am Ausgleichsstück 7 mit der Verzahnung 12 an der Lasche 9 zusammenwirken. Dies ist jedoch für die Funktion der Erfindung nicht notwendig.

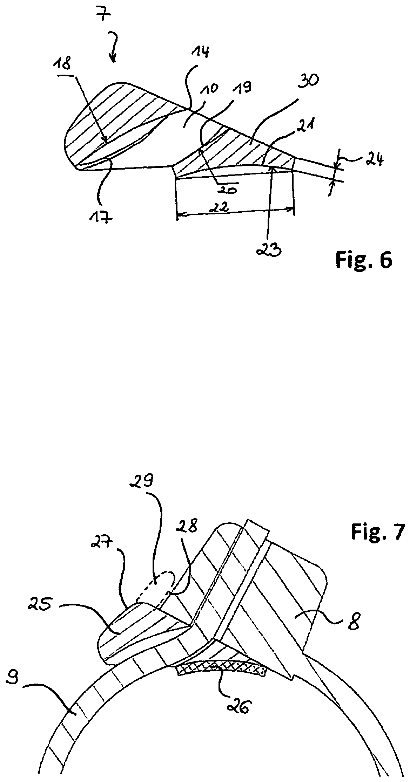

Bei dem in Figur 3 gezeigten Ausführungsbeispiel ist der von der Lasche 9 umgrenzte Hohlraum 15 kreisförmig mit einem Durchmesser 16 von $\frac{3}{8}$ Zoll. Abgestimmt auf dieses Ausführungsbeispiel zeigt die Figur 6 das Ausgleichsstück 7 mit einer konkav oder konvex ausgebildeten äußeren Anlagefläche 17 am Durchlass 10 mit einem Radius 18 von beispielsweise 10,7 cm. Die gegenüberliegende Fläche 19 ist konkav mit einem Radius 20 von beispielsweise 7,0 cm ausgebildet. Die dem Hohlraum 15 zugewandte Anlagefläche 21 hat eine Länge 22 von 4,6 mm und einen Radius 23 von 8,3 cm. Als Absatz 24 ist beidseitig der Anlagefläche 21 eine Höhe von 0,5 mm vorgesehen.

Die Figur 7 zeigt das Zusammenwirken des Kopfstückes 8 und der Lasche 9 mit einem Ausgleichsstück 25, das eine flexible Anlagefläche 26 aufweist, die vorzugsweise elastisch ausgebildet ist. Die Flexibilität sorgt für eine flächige Anlage, beispielsweise an einem im Hohlraum 15 angeordneten Schlauchstück (nicht gezeigt) und eine Elastizität wirkt einer Verringerung des Anpressdruckes bei einer Dehnung der Lasche 9 oder einem Schrumpfen des Schlauchstückes entgegen.

Eine vergrößerte Beschriftungsfläche 27 und eine zusätzliche Anlagefläche 28 des Ausgleichsstückes 25 am Kopfstück 8 wird durch den Vorsprung 29 am Ausgleichsstück erreicht.

Kumulativ oder alternativ zur flexiblen Fläche 26 kann auch im Bereich 30 zwischen dem Ausgleichsstück 7 und dem Kopfstück 8 oder im Bereich der Fläche 19 zwischen dem Ausgleichsstück 7 und der Lasche 9 eine flexible und vorzugsweise elastische Fläche (nicht gezeigt) vorgesehen sein, um langfristig einen ausreichenden Druck auf das vom Kabelbinder umgebenden Bauteil (nicht gezeigt) auszuüben.

Diese flexiblen Anlageflächen können auch als Absatz ausgebildet sein.

Das Ausgleichsstück 25 kann beispielsweise eine Höhe 31 von 3,5 mm, eine Länge 32 von 9, 1 mm und eine Breite B von 7 mm aufweisen. Der keilförmige Querschnitt wird von zwei langen Schenkeln 33, 34 und einer kurzen Basis 35 gebildet.

Der Übergang 36 von dem langen Schenkel 33 auf die kurze Basis 35 und der Übergang 37 von dem langen Schenkel 34 auf die kurze Basis 35 ist jeweils abgerundet ausgebildet. Die Anlagefläche 30 des langen Schenkels 33 für die Anlage am Kopfstück 8 ist hingegen plan.

Als Durchgang 10 für die Lasche 9 hat das Ausgleichsstück 25 einen Schlitz 38 dessen längere Seite 39 mit im Ausführungsbeispiel einer Breite B' von 5,5 mm der Breite 40 der Lasche 9 entspricht, sodass das Ausgleichsstück 25 auf der Lasche 9 verschiebbar ist und nicht lose von der Lasche 9 rutscht.

Die kürzere Seite 41 des Schlitzes 38 hat eine elastisch ausgebildete Anlagefläche, die gegen die Lasche 9 drückt und dadurch einen geringen Widerstand beim Verschieben des Ausgleichsstückes 25 auf der Lasche 9 bildet.

Die in Figur 6 gezeigte Kante 14 bildet einen Anschlag 42 an einer gezahnten äußeren Oberfläche 12 der Lasche 9 und wirkt als Gegenstück mit der Lasche 9 zusammen.

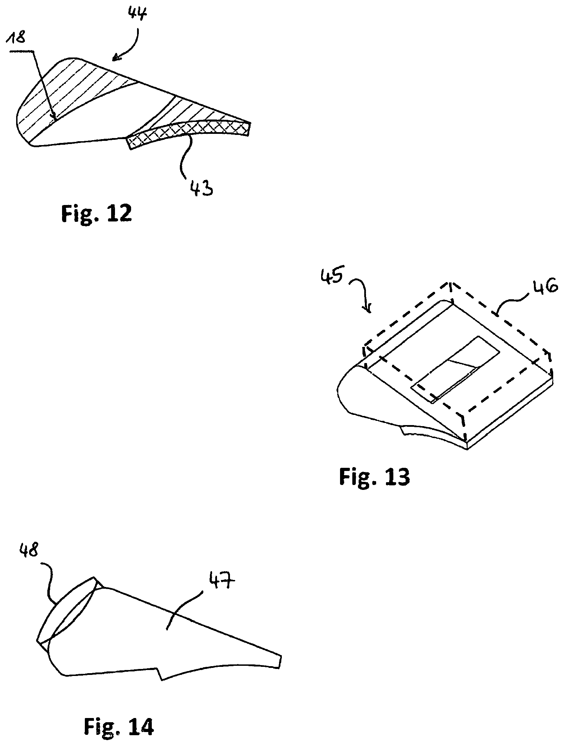

Die Figur 12 zeigt ein elastisches Teil 43 an dem Ausgleichsstück 44, das sich unter Druck verformen kann und für eine gute Anlage des Ausgleichstückes 44 an eine von der Lasche 9 umgrenzten Gegenstand sorgt.

Ein Ausgleichsstück 45 mit einer punktiert angedeuteten Erhöhung 46 als vorzugsweise elastisch ausgebildeter Anlagefläche des Ausgleichsstückes 45 an einem Kopf 8 des Kabelbinders ist in Figur 13 gezeigt.

Die Figur 14 zeigt ein Ausgleichsstück 47 mit einer vergrößerten Anlagefläche 48 an der kurzen Basis des Ausgleichsstückes. Diese Fläche ist für die Beschriftung oder die Anbringung eines Etikettes geeignet.

**Patentansprüche**

1. Kabelbinder mit einem Kopfstück und einer Lasche, die in das Kopfstück geschoben werden kann und sich dabei rastend fixiert, wobei der Kabelbinder ein Ausgleichsstück (5, 7, 25, 44) aufweist, das auf die Lasche aufgefädelt ist und das im Bereich des Kopfstücks (4, 8) positionierbar ist, um mit der Lasche (6, 9) einen Hohlraum (15) zu umgrenzen, und wobei das Ausgleichsstück in der Ebene der Umgrenzung des Hohlraumes (15) einen keilförmigen Querschnitt mit zwei langen Schenkeln (33, 34) und einer kurzen Basis (35) aufweist, *dadurch gekennzeichnet, dass* das Ausgleichsstück (5, 7, 25, 44) eine einen langen Schenkel (33) bildende Fläche aufweist, die als Anlage für das Kopfstück (8) plan ausgebildet ist.

2. Kabelbinder nach Anspruch 1, *dadurch gekennzeichnet, dass* das Ausgleichsstück (5, 7, 25) an seiner dem Hohlraum (15) zugewandten Seite eine konkave Oberfläche (21) aufweist.

3. Kabelbinder nach Anspruch 2, *dadurch gekennzeichnet, dass* die Lasche einen kreisförmigen Hohlraum umgrenzt und die konkave Oberfläche (21) etwa dem Radius (23) des kreisförmigen Hohlraums (15) entspricht.

4. Kabelbinder nach einem der Ansprüche 2 oder 3, *dadurch gekennzeichnet, dass* zwischen der konkaven Oberfläche (21) und dem Kopfstück (4, 8) ein Absatz vorgesehen ist.

5. Kabelbinder nach einem der Ansprüche 2 bis 4, *dadurch gekennzeichnet, dass* zwischen der konkaven Oberfläche (21) und der Lasche (9) ein Absatz vorgesehen ist.

6. Kabelbinder nach einem der vorhergehenden Ansprüche, *dadurch gekennzeichnet, dass* der Übergang (36, 37) von der einen langen Schenkel (33, 34) bildenden Fläche zu der die Basis (35) bildenden Fläche abgerundet ist.

7. Kabelbinder nach einem der vorhergehenden Ansprüche, *dadurch gekennzeichnet, dass* das Ausgleichsstück (5, 7, 25, 44) einen rechteckigen Schlitz (38) für die Aufnahme der Lasche (9) aufweist, dessen längere Seite (39) der Breite (40) der Lasche (9) entspricht, sodass es auf der Lasche (9) verschiebbar ist und nicht lose aus der Lasche (9) rutscht.

8. Kabelbinder nach einem der vorhergehenden Ansprüche, *dadurch gekennzeichnet, dass* das Ausgleichsstück (5, 7, 25, 44) einen rechteckigen Schlitz (38) für die Aufnahme der Lasche (9) aufweist, dessen kürzere oder längere Seite (39, 41) eine federnd oder elastisch ausgebildete Anlagefläche aufweist, sodass diese Anlagefläche gegen die Lasche (9) drückt.

9. Kabelbinder nach einem der vorhergehenden Ansprüche, *dadurch gekennzeichnet, dass* das Ausgleichsstück (5, 7, 25; 44) einen Anschlag (42) aufweist, der mit einem Gegenstück an der Lasche (9) zusammenwirkt.

10. Kabelbinder nach einem der vorhergehenden Ansprüche, *dadurch gekennzeichnet, dass das* Ausgleichsstück (44) ein elastisches Teil (43) ist oder aufweist, das sich unter Druck verformt.

11. Kabelbinder nach einem der vorhergehenden Ansprüche, *dadurch gekennzeichnet, dass* das Ausgleichsstück (5, 7, 25, 44) ein Spritzgussteil und vorzugsweise ein Zweikomponentenspritzgussteil oder ein im 3D Druck hergestelltes Teil ist.

**12.** Set aus einem Kabelbinder nach einem der vorhergehenden Ansprüche und einem Schlauchstück.

**Claims**

**1.** A cable tie having a head piece and a strap, which can be pushed into the head piece, thereby fixing itself in a latching manner, the cable tie having a compensation piece (5, 7, 25, 44), which is threaded onto the strap and which can be positioned in the region of the head piece (4, 8) in order to bound a hollow space (15) together with the strap (6, 9), and wherein the compensation piece has a wedge-shaped cross section in the plane of the perimeter of the hollow space (15), with two long arms (33, 34) and a short base (35), **characterized in that** the compensation piece (5, 7, 25, 44) has a surface, which forms a long arm (33) and is designed planar as an abutment for the head piece (8).

**2.** The cable tie according to Claim 1, **characterized in that** the compensation piece (5, 7, 25) has a concave surface (21) on its side which faces the hollow space (15).

**3.** The cable tie according to Claim 2, **characterized in that** the strap bounds a circular hollow space, and the concave surface (21) approximately corresponds to the radius (23) of the circular hollow space (15).

**4.** The cable tie according to any one of Claims 2 or 3, **characterized in that** a shoulder is provided between the concave surface (21) and the head piece (4, 8).

**5.** The cable tie according to any one of Claims 2 to 4, **characterized in that** a shoulder is provided between the concave surface (21) and the strap (9).

**6.** The cable tie according to any one of the preceding claims, **characterized in that** the transition (36, 37) from the surface which forms a long arm (33, 34) is rounded off towards the surface which forms the base (35).

**7.** The cable tie according to any one of the preceding claims, **characterized in that** the compensation piece (5, 7, 25, 44) has a rectangular slot (38) for receiving the strap (9), the longer side (39) of which corresponds to the width (40) of the strap (9), so that it can be displaced on the strap (9) and does not loosely slip off the strap (9).

**8.** The cable tie according to any one of the preceding claims, **characterized in that** the compensation piece (5, 7, 25, 44) has a rectangular slot (38) for receiving the strap (9), the shorter or longer side (39, 41) of which has a resilient or elastic abutment surface, so that this abutment surface presses against the strap (9).

**9.** The cable tie according to any one of the preceding claims, **characterized in that** the compensation piece (5, 7, 25, 44) has a stop (42), which interacts with a counterpart on the strap (9).

**10.** The cable tie according to any one of the preceding claims, **characterized in that** the compensation piece (44) is or has an elastic part (43), which deforms under compression.

**11.** The cable tie according to any one of the preceding claims, **characterized in that** the compensation piece (5, 7, 25, 44) is an injection-moulded part and preferably a two-component injection-moulded part or a part manufactured by 3D printing.

**12.** Set consisting of a cable tie according to any one of the preceding claims and a piece of tubing.

**Revendications**

**1.** Collier de serrage comprenant un élément faisant office de tête et une patte qui peut être insérée dans l'élément faisant office de tête en s'y fixant par encliquetage, dans lequel le collier de serrage présente un élément de compensation (5, 7, 25, 44) qui est enfilé sur la patte et qui peut être positionné, dans la zone de l'élément faisant office de tête (4, 8), de façon à délimiter avec la patte (6, 9) un espace creux (15), et dans lequel l'élément de compensation présente, dans le plan de la délimitation de l'espace creux (15), un tronçon de forme conique comprenant deux grandes branches (33, 34) et une petite base (35), **caractérisé en ce que** l'élément de compensation (5, 7, 25, 44) présente une surface qui forme une grande branche (33), qui est réalisée sous forme plane pour servir d'appui pour l'élément faisant office de tête (8).

**2.** Collier de serrage selon la revendication 1, **caractérisé en ce que** l'élément de compensation (5, 7, 25) présente, sur son côté tourné vers l'espace creux (15), une surface concave (21).

**3.** Collier de serrage selon la revendication 2, **caractérisé en ce que** la patte délimite un espace creux de forme circulaire et la surface concave (21) correspond approximativement au rayon (23) de l'espace creux (15) de forme circulaire.

**4.** Collier de serrage selon l'une quelconque des revendications 2 ou 3, **caractérisé en ce que**, entre la surface concave (21) et l'élément faisant office de tête (4, 8), on prévoit un épaulement.

5. Collier de serrage selon l'une quelconque des revendications 2 à 4, **caractérisé en ce que**, entre la surface concave (21) et la patte (9), on prévoit un épaulement.

6. Collier de serrage selon l'une quelconque des revendications précédentes, **caractérisé en ce que** la transition (36, 37) de la surface formant une grande branche (33, 34) à la surface formant la base (35) est arrondie.

7. Collier de serrage selon l'une quelconque des revendications précédentes, **caractérisé en ce que** l'élément de compensation (5, 7, 25, 44) présente une fente rectangulaire (38) pour la réception de la patte (9), dont le plus grand côté (39) correspond à la largeur (40) de la patte, si bien qu'il peut se déplacer sur la patte (9) et qu'il ne se désolidarise pas de la patte (9) par glissement.

8. Collier de serrage selon l'une quelconque des revendications précédentes, **caractérisé en ce que** l'élément de compensation (5, 7, 25, 44) présente une fente rectangulaire (38) pour la réception de la patte (9), dont le plus petit ou le plus grand côté (39, 41) présente une surface d'appui réalisée de manière à faire ressort ou de manière élastique, d'une manière telle que cette surface d'appui exerce une pression sur la patte (9).

9. Collier de serrage selon l'une quelconque des revendications précédentes, **caractérisé en ce que** l'élément de compensation (5, 7, 25 ; 44) présente une butée (42) qui coopère avec un élément antagoniste contre la patte (9).

10. Collier de serrage selon l'une quelconque des revendications précédentes, **caractérisé en ce que** l'élément de compensation (44) est ou comprend une partie élastique (43) qui se déforme sous pression.

11. Collier de serrage selon l'une quelconque des revendications précédentes, **caractérisé en ce que** l'élément de compensation (5, 7, 25, 44) représente une pièce moulée par injection et de préférence une pièce moulée par injection du type à deux composants ou une pièce fabriquée par impression 3D.

12. Ensemble constitué par un collier de serrage selon l'une quelconque des revendications précédentes et par un élément en forme de tuyau flexible.

**Fig. 1**

**Fig. 2**

**Fig. 3**

8

7

40

9

**Fig. 4**

8

7

9

**Fig. 5**

Fig. 6

Fig. 7

Fig. 8

Fig. 9

Fig. 10

Fig. 11

**Fig. 12**

**Fig. 13**

**Fig. 14**

**IN DER BESCHREIBUNG AUFGEFÜHRTE DOKUMENTE**

**In der Beschreibung aufgeführte Patentdokumente**

- EP 2247882 B1